# EUROPÄISCHE PATENTSCHRIFT

(11) **EP 0 718 260 B1**
(45) Veröffentlichungstag und Bekanntmachung des Hinweises auf die Patenterteilung: **12.08.1998**
(21) Anmeldenummer: 95119455.4
(22) Anmeldetag: 11.12.1995
(51) Int. Cl.: C07C 17/087, C07B 39/00, C07C 209/86

(54) **Verfahren zur Aufarbeitung von komplexen Aminhydrofluoriden**
Method for working up complex amine hydrofluorides
Procédé pour le retraitement des complexes des aminehydrofluorides

(30) Priorität: 20.12.1994 DE 4445529
(43) Veröffentlichungstag der Anmeldung: 26.06.1996
(73) Patentinhaber: SOLVAY (Société Anonyme), B-1050 Bruxelles (BE)
(72) Erfinder: Hahn, Ulrich, D-60322 Frankfurt (DE); Franz, Raimund, Dr., D-65779 Kelkheim (DE); Siegemund, Günter, Dr., D-65719 Hofheim (DE)
(74) Vertreter: Jacques, Philippe

(56) Entgegenhaltungen:
- EP-A- 0 634 383
- WO-A-86/00294

## Beschreibung

Die vorliegende Erfindung betrifft ein Aufarbeitungsverfahren für komplexe Amin-Hydrofluoride, die zur Addition von Fluorwasserstoff an halogenierte Alkene verwendet werden.

In der Deutschen Patentanmeldung P 43 39 539.2 wird ein Verfahren zur Addition von Fluorwasserstoff an halogenierte Alkene mittels flüssiger komplexer Hydrofluoride von organischen Stickstoffbasen beschrieben. Vorzugsweise werden dabei komplexe Hydrofluoride der Formel [A • n HF] mit 2 < n < 3 eingesetzt, in der A für ein tertiäres Amin steht. Hydrofluoride dieser Art sind z.B. aus J. Fluorine Chemistry 15 (1980), Seite 423-434 bekannt und werden sehr leicht durch Umsetzung geeigneter Amine mit einer dieser Formel entsprechenden Menge Fluorwasserstoff erhalten. Sie setzen sich mit halogenierten Alkenen, wie z.B. Hexafluorpropen oder Trifluorchlorethylen, vorzugsweise bei Temperaturen von 0 bis 100°C um, wobei eine Addition von Fluorwasserstoff an die halogenierten Alkene eintritt. Nach der destillativen Isolierung des Additionsproduktes kann durch Umsetzung des Reaktionsrückstandes mit Fluorwasserstoff die ursprüngliche Zusammensetzung des komplexen Hydrofluorids wiederhergestellt und die Additionsreaktion somit beliebig oft wiederholt werden.

In der oben erwähnten Patentanmeldung ist auch eine kontinuierliche Durchführung der Addition von HF an gasförmige halogenierte Alkene in einer Blasensäule unter simultaner Zufuhr äquivalenter Mengen an halogeniertem Alken und Fluorwasserstoff zum flüssigen komplexen Hydrofluorid beschrieben. Dieses kann daher als stets wiederverwendbarer Katalysator für die Umsetzung geeigneter halogenierter Alkene mit Fluorwasserstoff betrachtet und verwendet werden.

Bei der Verwendung von Fluorwasserstoff "technischer" Qualität in dem genannten Prozeß reichern sich allerdings im Laufe der Zeit die darin enthaltenen Verunreinigungen im komplexen Hydrofluorid an und können schließlich den Umsatz und die Produktqualität beeinträchtigen. Zu diesen Verunreinigungen zählen vor allem Schwefeldioxid, Schwefelsäure und Fluokieselsäure, die sich mit komplexen Hydrofluoriden zu nichtflüchtigen Verbindungen umsetzen. Der flüssige Reaktorinhalt muß daher in bestimmten Zeitabständen ausgewechselt und möglichst umweltfreundlich aufgearbeitet werden; eine solche Aufarbeitung kann jedoch auch aus anderen Gründen notwendig erscheinen, z. B. bei Stillegung einer Produktionsanlage.

Es wurde nun bei der Durchführung der oben erwähnten Umsetzung von Amin-Hydrofluoriden mit halogenierten Alkenen überraschenderweise gefunden, daß im Fall der Verwendung komplexer Hydrofluoride bestimmter tertiärer Amine mit kurzkettigen Alkylresten, wie z.B. [n-C₃-H₇)₃N•2,9 HF] oder [(n-C₄H₉)₃N•2,6 HF], ein Absinken des HF/Amin-Molverhältnisses auf etwa 1,5 bis 2 dazu führt, daß sich eine zweite, spezifisch leichtere, flüssige Phase abscheidet, die aus praktisch reinem Amin besteht. Die Menge des komplexen Hydrofluorids nimmt im weiteren Verlauf der Umsetzung zugunsten der neugebildeten Aminphase ab, was zu einer Anreicherung der gelösten Verunreinigungen in der Hydrofluoridphase führt. Man kann auf diese Weise eine vorteilhafte Aufarbeitung des komplexen Hydrofluorids durchführen, denn das sich abscheidende Amin kann abgetrennt und wiederverwendet werden. Die mit Verunreinigungen angereicherte Hydrofluorid-Phase hingegen kann vernichtet und der Verlust an Amin und Fluorwasserstoff damit auf ein Minimum reduziert werden.

Gegenstand der vorliegenden Erfindung ist somit ein Verfahren zur Aufarbeitung eines flüssigen komplexen Hydrofluorids eines tertiären Amins der allgemeinen Formel (I)

[R¹ R² R³ N • n HF] (I)

worin
die Reste R¹, R² und R³ gleich oder verschieden sind und geradkettige oder verzweigte C₁-C₈-Alkylgruppen bedeuten, die zusammen mindestens 7 C-Atome haben und
n eine ganze oder gebrochene Zahl mit 1,5 < n < 3 bedeutet,
das zwecks Addition von HF an ein halogeniertes Alken der allgemeinen Formel (II)

R⁴CF=CR⁵R⁶ (II)

R⁴ = F, CF₃ oder C₂F₅
R⁵ = F, Cl oder CF₃
R⁶ = H, F, oder perfluoriertes C₁-C₄-Alkyl
mit diesem halogenierten Alken umgesetzt wird, dadurch gekennzeichnet, daß man bei der Umsetzung des halogenierten Alkens der Formel (II) mit dem komplexen Amin-Hydrofluorid der Formel (I) das Molverhältnis HF : R¹R²R³N soweit absinken läßt, bis sich Amin in einer weiteren flüssigen Phase abscheidet, und das Amin abtrennt.

Welcher Prozentsatz des im komplexen Hydrofluorid (I) [R¹R²R³N•nHF] enthaltenen Amins sich abscheidet, hängt davon ab, welcher Prozentsatz des in (I) enthaltenen HF durch Addition an das halogenierte Alken (II) verbraucht wird. Den Prozentsatz des verbrauchten HF kann man durch Einstellung eines geeigneten Mengenverhältnisses von (I):(II) steuern. Bei diskontinuierlicher Arbeitsweise wird dieses Mengenverhältnis vor Beginn der Umsetzung eingestellt. Bei kontinuierlicher Arbeitsweise wird das genannte Mengenverhältnis während der Umsetzung eingestellt, nämlich indem man nur das verbrauchte halogenierte Alken (II) ersetzt, aber nicht das verbrauchte HF.

Im allgemeinen läßt man 5 bis 90 % des Amins sich abscheiden, vorzugsweise 10 bis 90 %. Es ist ohne weiteres möglich, 100 % des Amins sich abscheiden zu lassen, indem man geeignete Mengenverhältnisse von halogeniertem Alken (II):Hydrofluorid (I) vor der diskontinuierlichen Umsetzung oder während der kontinuierlichen Umsetzung einstellt. Eine Abscheidung von 100 % des genannten Amins ist aber häufig nicht von Interesse.

Das vorliegende Aufarbeitungsverfahren ist außerordentlich überraschend. Jedem Fachmann ist zwar bekannt, daß Hydrohalogenide des Ammoniaks und organischer Amine in Wasser löslich sind und daß durch Umsetzung dieser wäßrigen Lösungen mit Lauge, z.B. Natronlauge, der Ammoniak oder das Amin freigesetzt werden kann. Die Addition des in den komplexen Amin-Hydrofluoriden (I) enthaltenen Fluorwasserstoffs an halogenierte Alkene (II) wird jedoch nicht in Wasser durchgeführt. Es war daher keineswegs zu erwarten, daß beim Verbrauch des HF sogar lange vor Erreichen des stöchiometrischen HF/Amin-Verhältnisses von 1 : 1, die Abscheidung einer weiteren, flüssigen, aus reinem Amin bestehenden Phase erfolgt.

Die komplexen Amin-Hydrofluoride der Formel (I) sind vorzugsweise von folgenden tertiären Aminen R¹R²R³N abgeleitet: Tri-n-propylamin, Tri-n-butylamin, Tri-n-pentylamin, Tri-isopentylamin, Tri-n-hexylamin, Methyl-n-propyl-n-butylamin, n-butylamin, Methyl-isopropyl-n-butylamin. Besonders bevorzugt ist hierbei

Als halogenierte Alkene der Formel (II) setzt man insbesondere die folgenden ein:
CF₂ = CF₂, CF₂ = CCIF, CF₂ = CF-CF₃, CF₃-CF = CH-CF₃, CF₃-CF =C(CF₃)₂,
C₂F₅-CF=C(CF₃)₂, C₂F₅-CF=C(CF₃)(C₄F₉),
vor allem aber CF₂=CF-CF₃ (Hexafluorpropen).

Aus diesen halogenierten Alkenen enstehen dabei durch die Addition von HF folgende halogenierte Alkane:

| Alken (II) | Alkan |
|---|---|
| CF₂=CF₂ | CF₃-CF₂H |
| CF₂=CClF | CF₃-CHClF |
| CF₂=CF-CF₃ | CF₃-CHF-CF₃ |
| CF₃-CF=CH-CF₃ | CF₃-CF₂-CH₂-CF₃ |
| CF₃-CF=C(CF₃)₂ | CF₃-CF₂-CH(CF₃)₂ |
| C₂F₅-CF=C(CF₃)₂ | C₂F₅-CF₂-CH(CF₃)₂ |
| C₂F₅-CF=C(CF₃)(C₄F₉) | C₂F₅-CF₂-CH(CF₃)(C₄F₉) |

Man kann auch ein Gemisch aus zwei oder mehr halogenierten Alkenen der Formel (II) einsetzen.

Die erfindungsgemäße Aufarbeitung der zur Addition von HF an halogenierte Alkene (II) verwendeten komplexen Hydrofluoride (I) kann unter Bedingungen und in Apparaturen durchgeführt werden, die denen der Additionsreaktion gleichen oder ähnlich sind. Die Temperaturen betragen in Abhängigkeit von dem eingesetzten komplexen Hydrofluorid im allgemeinen 0 bis 150°C, vorzugsweise 10 bis 90°C, besonders bevorzugt 60 bis 80°C.

Die Art der Apparatur richtet sich danach, ob die Aufarbeitung diskontinuierlich oder kontinuierlich durchgeführt werden soll, sowie nach den Siedepunkten des eingesetzten halogenierten Alkens (II) und des daraus durch HF-Addition entstehenden halogenierten Alkans und nach der Reaktivität des halogenierten Alkens.

Bei diskontinuierlicher Durchführung können beispielsweise mit Rückflußkühler versehene Rührbehälter verwendet werden, die für den Labor- oder Technikumsmaßstab auch aus Borsilikatglas bestehen können. Beim Einsatz tiefsiedender halogenierter Alkene (II) wird der Aufarbeitungsprozeß zweckmäßig in geschlossenen Druckbehältern mit Rührwerk durchgeführt. Man wählt bei der diskontinuierlichen Durchführung der Aufarbeitung das Molverhältnis halogeniertes Alken (II): komplexes Amin-Hydrofluorid (I) zu Beginn ihrer Umsetzung im allgemeinen so, daß sich 5 bis 90 % des im Hydrofluorid (I) enthaltenen Amins, vorzugsweise aber 10 bis 90 % des Amins, abscheiden. Nach der Isolierung des bei der HF-Addition entstehenden halogenierten Alkans wird die abgeschiedene Aminphase abgetrennt und kann erneut in das komplexe Hydrofluorid (I) umgewandelt werden.

Die kontinuierliche Durchführung der HF-Addition an ein gasförmiges halogeniertes Alken (II) wird, wie einleitend erwähnt, unter simultaner Zufuhr von halogeniertem Alken und Fluorwasserstoff zu dem komplexen Hydrofluorid (I) vorgenommen, z.B. in einer Blasensäule. Zur Aufarbeitung des komplexen Hydrofluorids wird unter weiterer Zufuhr von halogeniertem Alken die Zufuhr von Fluorwasserstoff unterbrochen. Nachdem sich das Amin als separate Phase abgeschieden hat, kann dieses (diskontinuierlich) entnommen werden. Die Aufarbeitung kann auch kontinuierlich gestaltet werden, indem nach Unterbrechung der HF-Zufuhr komplexes Hydrofluorid (I) in die Apparatur eingespeist und das sich abscheidende Amin im Maße seiner Bildung kontinuierlich entnommen wird.

Die Verwendung von Blasensäulen erlaubt auch eine simultane Durchführung der HF-Addition und der Aufarbeitung des komplexen Hydrofluorids. Dies kann zum Beispiel dadurch erreicht werden, daß aus der Blasensäule, in der die HF-Addition abläuft, kontinuierlich ein kleiner Teil des komplexen Hydrofluorids entnommen und in einer sehr viel kleineren Apparatur aufgearbeitet wird, oder dadurch, daß zwei Blasensäulen ähnlicher Größe eine Kaskade bilden, wobei das gasförmige, halogenierte Alken zunächst in die zur Aufarbeitung benutzte erste Blasensäule eintritt und das dort entstehende Gasgemisch dann in die zur HF-Addition benutzte zweite Blasensäule eingeleitet wird.

Die Erfindung wird durch die im folgenden angeführten Beispiele erläutert. Die Prozentzahlen sind Gewichtsprozente falls nichts anders angegeben oder offensichtlich gemeint ist.

### Beispiel 1:

In einem mit Rührer ausgestatteten Autoklaven von 300 ccm Fassungsvermögen wurden 90 g (0,4 Mol) eines bei der Addition von Fluorwasserstoff an Hexafluorpropen während 800 Betriebsstunden verwendeten komplexen Hydrofluorids des Tri-n-butylamins vorgelegt. Das Hydrofluorid war homogen und flüssig und wies ein HF/Amin-Verhältnis von n = 2,1 auf. Nach dem Verschließen des Autoklaven wurden 60 g Hexafluorpropen (0,4 Mol) aus einem Vorrats-Druckbehälter eingedrückt und das Reaktionsgemisch bei 50°C über Nacht gerührt. Anschließend wurden nach Entspannen des Autoklaven die tiefsiedenden Anteile in einer mit Trockeneis gekühlten Falle kondensiert. Die Ausbeute an rohem Heptafluorpropan betrug 63,3 g (93 % der Theorie), mit einer Reinheit (laut Gaschromatogramm) von 95 %. Der flüssige Rückstand im Autoklaven wog 83 g und bestand aus zwei Phasen, die in einem Scheidetrichter getrennt wurden. Die leichtere Phase bestand aus 31 g Tri-n-butylamin, das laut Gaschromatogramm eine Reinheit von 98,8 % aufwies; als schwerere Phase wurden 52 g an komplexem Hydrofluorid mit einem HF/Amin-Verhältnis von n = 1,9 zurückerhalten.

### Beispiel 2:

In einem gläsernen Reaktionskolben von 250 ccm Fassungsvermögen mit Rückflußkühler wurden 45 g (0,2 Mol) eines komplexen Hydrofluorids des Tri-n-butylamins vorgelegt, das ein HF/Amin-Verhältnis von n = 2 aufwies. Dann wurden 60 g Perfluor-(2-methyl-2-penten) (0,2 Mol) zugetropft und das so entstandene Gemisch 6 Stunden bei 50 bis 60°C gerührt. Das durch Addition von HF an das Fluorpenten entstandene rohe 2-Trifluormethyl-2-H-decafluor-n-pentan wurde anschließend bei einer Siedetemperatur zwischen 60 und 65°C abdestilliert. Die Ausbeute betrug 60 g (93,8 % der Theorie). Der Destillationsrückstand bestand aus zwei flüssigen Phasen, die in einem Scheidetrichter getrennt wurden. Die leichtere Phase bestand aus 18 g Tri-n-butylamin, das laut Gaschromatogramm eine Reinheit von 98 % aufwies; als schwerere Phase wurden 20 g an komplexem Hydrofluorid mit einem HF/Amin-Verhältnis von n = 2 zurückerhalten.

### Beispiel 3:

Ein komplexes Hydrofluorid des Tri-n-butylamins, das 1200 Std. als Medium und Reagens für die Addition von Fluorwasserstoff an Hexafluorpropen benutzt worden war, wies nunmehr Verunreinigungen auf, die gemäß Elementaranalyse einem Gehalt an Schwefel von 0,1 % und von Silicium von 0,03 % entsprachen. Das HF/Amin-Verhältnis in dem benutzten komplexen Hydrofluorid (I) betrug 2 : [(n-C₄H₉)₃N • 2 HF]. Von diesem Hydrofluorid wurden 200 g (0,89 Mol) in eine von außen beheizbare Blasensäule aus Borsilikatglas von 70 cm Länge und 22 mm lichter Weite gefüllt und auf 60°C erwärmt. Die Flüssigkeitshöhe des Hydrofluorids betrug zu diesem Zeitpunkt 60 cm. Am Boden der Säule wurde gasförmiges Hexafluorpropen eingeleitet und fein verteilt. An seiner hellen Färbung leicht erkennbar, schied sich aus dem komplexen Hydrofluorid alsbald Tri-n-butylamin als leichtere, zweite flüssige Phase ab, deren Volumen auf Kosten der Hydrofluoridphase zunahm. Am Kopf der Säule trat das durch Addition von HF an das Hexafluorpropen entstandene rohe Heptafluorpropan (Rohgas) aus und wurde in einer Kühlfalle kondensiert. Dem Strom des Rohgases wurden von Zeit zu Zeit Proben für die gaschromatographische Analyse entnommen.

Bei einer Flüssigkeitshöhe der Hydrofluoridphase von 6 cm (entsprechend 10 % des anfänglichen Werts von 60 cm) wurde eine Probe dieser Phase entnommen und analysiert; der Gehalt an Schwefel betrug nunmehr 1,2 % und an Silicium 0,27 %. Der Umsatz des Hexafluorpropens betrug selbst bei dieser geringen Flüssigkeitshöhe noch über 50 %. Das als zweite flüssige Phase abgeschiedene Tri-n-butylamin (140 g) wies eine nur schwach gelbe Färbung und eine Reinheit von 98 % auf.

## Patentansprüche

1. Verfahren zur Aufarbeitung eines flüssigen komplexen Hydrofluorids eines tertiären Amins der allgemeinen Formel (I)
[R¹ R² R³ N • n HF] (I)
worin
die Reste R¹, R² und R³ gleich oder verschieden sind und geradkettige oder verzweigte C₁-C₈-Alkylgruppen bedeuten, die zusammen mindestens 7 C-Atome haben und
n eine ganze oder gebrochene Zahl mit 1,5 < n < 3 bedeutet
das zwecks Addition von HF an ein halogeniertes Alken der allgemeinen Formel (II)
R⁴CF=CR⁵R⁶ (II)
R⁴ = F, CF₃ oder C₂F₅
R⁵ = F, Cl oder CF₃
R⁶ = H, F, oder perfluoriertes C₁-C₄-Alkyl
mit diesem halogenierten Alken umgesetzt wird, dadurch gekennzeichnet, daß man bei der Umsetzung des halogenierten Alkens der Formel (II) mit dem komplexen Amin-Hydrofluorid der Formel (I) das Molverhältnis HF : R¹R²R³N soweit absinken läßt, bis sich Amin in einer weiteren flüssigen Phase abscheidet, und das Amin abtrennt.

2. Verfahren nach Anspruch 1, dadurch gekennzeichnet, daß ein komplexes Hydrofluorid der Formel (I) aufgearbeitet wird, bei dem das Amin R¹R²R³N Tri-n-propylamin, Tri-n-butylamin, Tri-n-pentylamin, Tri-isopentylamin, Tri-n-hexylamin, Methyl-n-propyl-n-butylamin oder Methyl-isopropyl-n-butylamin ist.

3. Verfahren nach Anspruch 1, dadurch gekennzeichnet, daß ein komplexes Hydrofluorid der Formel (I) aufgearbeitet wird, bei dem das Amin R¹R²R³N Tri-n-butylamin ist.

4. Verfahren nach einem der Ansprüche 1 bis 3, dadurch gekennzeichnet, daß die Aufarbeitung bei einer Temperatur von 0 bis 150°C durchgeführt wird.

5. Verfahren nach einem der Ansprüche 1 bis 3, dadurch gekennzeichnet, daß die Aufarbeitung bei einer Temperatur von 10 bis 90°C durchgeführt wird.

6. Verfahren nach einem der Ansprüche 1 bis 3, dadurch gekennzeichnet, daß die Aufarbeitung bei einer Temperatur von 60 bis 80°C durchgeführt wird.

7. Verfahren nach einem der Ansprüche 1 bis 6, dadurch gekennzeichnet, daß man das komplexe Hydrofluorid (I) diskontinuierlich mit dem halogenierten Alken (II) umsetzt und dabei das Molverhältnis halogeniertes Alken (II): komplexes Amin-Hydrofluorid (I) zu Beginn ihrer Umsetzung so wählt, daß sich 5 bis 90 % des im Hydrofluorid (I) enthaltenen Amins als weitere flüssige Phase abscheiden, und das Amin abtrennt.

8. Verfahren nach Anspruch 7, dadurch gekennzeichnet, daß man das Molverhältnis halogeniertes Alken (II): komplexes Amin-Hydrochlorid (I) so wählt, daß sich 10 bis 90 % des Amins abscheiden.

9. Verfahren nach einem der Ansprüche 1 bis 6, dadurch gekennzeichnet, daß man die Aufarbeitung des komplexen Hydrofluorids (I) durch Umsetzung mit einem gasförmigen halogenierten Alken (II) in einem schlanken Reaktor, z.B. einer Blasensäule, durchführt und das als separate Phase abgeschiedene Amin abtrennt.

10. Verfahren nach Anspruch 9, dadurch gekennzeichnet, daß man kontinuierlich das sich abscheidende Amin im Maße seiner Bildung abtrennt und durch eine äquivalente Menge an komplexem Amin-Hydrofluorid (I) ersetzt.

## Claims

1. A process for the work-up of a liquid complex hydrofluoride of a tertiary amine of the formula (I)
[R¹R²R³N•n HF] (I)
where
the radicals R¹, R² and R³ are identical or different and are straight-chain or branched C₁-C₈-alkyl groups which together have at least 7 carbon atoms and
n is an integer or fractional number where 1.5 < n < 3,
which, for the purpose of molecular addition of HF to a halogenated alkene of the formula (II)
R⁴CF=CR⁵R⁶ (II)
R⁴ = F, CF₃ or C₂F₅
R⁵ = F, Cl or CF₃
R⁶ = H, F or perfluorinated C₁-C₄-alkyl,
is reacted with this halogenated alkene, which comprises, in the reaction of the halogenated alkene of the formula (II) with the complex amine hydrofluoride of the formula (I), allowing the molar ratio HF:R¹R²R³N to fall until amine separates out as a further liquid phase, and separating off the amine.

2. The process as claimed in claim 1, wherein the work-up is carried out on a complex hydrofluoride of the formula (I) in which the amine R¹R²R³N is tri-n-propylamine, tri-n-butylamine, tri-n-pentylamine, triisopentylamine, tri-n-hexylamine, methyl-n-propyl-n-butylamine or methylisopropyl-n-butylamine.

3. The process as claimed in claim 1, wherein the work-up is carried out on a complex hydrofluoride of the formula (I) in which the amine R¹R²R³N is tri-n-butylamine.

4. The process as claimed in any one of claims 1 to 3, wherein the work-up is carried out at a temperature of from 0 to 150°C.

5. The process as claimed in any one of claims 1 to 3, wherein the work-up is carried out at a temperature of from 10 to 90°C.

6. The process as claimed in any one of claims 1 to 3, wherein the work-up is carried out at a temperature of from 60 to 80°C.

7. The process as claimed in any one of claims 1 to 6, wherein the complex hydrofluoride (I) is reacted batchwise with the halogenated alkene (II) and the molar ratio of halogenated alkene (II): complex amine hydrofluoride (I) at the beginning of their reaction is selected so that from 5 to 90 % of the amine present in the hydrofluoride (I) separates out as a further liquid phase, and the amine is separated off.

8. The process as claimed in claim 7, wherein the molar ratio of halogenated alkene (II): complex amine hydrofluoride (I) is selected so that from 10 to 90 % of the amine separates out.

9. The process as claimed in any one of claims 1 to 6, wherein the work-up of the complex hydrofluoride (I) is carried out by reaction with a gaseous halogenated alkene (II) in a slender reactor, e.g. a bubble column, and the amine which separates out as a separate phase is separated off.

10. The process as claimed in claim 9, wherein the amine which separates out is separated off continuously at the rate at which it is formed and is replaced by an equivalent amount of complex amine hydrofluoride (I).

## Revendications

1. Procédé pour le traitement d'un fluorhydrate complexe liquide d'une amine tertiaire répondant à la formule générale (I)
[R¹ R² R³ N . n HF] (I)
dans laquelle
les radicaux R¹, R² et R³ sont identiques ou différents et représentent des groupes alkyle en C₁-C₈ à chaînes droites ou ramifiées qui contiennent ensemble au moins 7 atomes de carbone, et
n représente un entier ou un nombre fractionnaire en respectant l'équation 1,5 < n < 3,
que l'on fait réagir avec l'alcène halogéné en vue d'addition de HF sur un alcène halogéné répondant à la formule générale (II)
R⁴CF=CR⁵R⁶ (II)
dans laquelle
R⁴ représente F, CF₃ ou C₂F₅,
R⁵ représente F, Cl ou CF₃,
R⁶ représente H, F ou encore un groupe alkyle en C₁-C₄ perfluoré,
caractérisé en ce que, lors de la mise en réaction de l'alcène halogéné de formule (II) avec le fluorhydrate d'amine complexe de formule (I), on laisse s'abaisser le rapport molaire HF:R¹R²R³N jusqu'à ce que l'amine se sépare en une phase liquide supplémentaire et on sépare l'amine.

2. Procédé selon la revendication 1, caractérisé en ce qu'on traite un fluorhydrate complexe de formule (I), dans lequel l'amine R¹R²R³N représente la tri-n-propylamine, la tri-n-butylamine, la tri-n-pentylamine, la tri-isopentylamine, la tri-n-hexylamine, la méthyl-n-propyl-n-butylamine ou la méthyl-isopropyl-n-butylamine.

3. Procédé selon la revendication 1, caractérisé en ce qu'on traite un fluorhydrate complexe de formule (I), dans lequel l'amine R¹R²R³N représente la tri-n-butylamine.

4. Procédé selon l'une quelconque des revendications 1 à 3, caractérisé en ce qu'on effectue le traitement à une température de 0 à 150°C.

5. Procédé selon l'une quelconque des revendications 1 à 3, caractérisé en ce qu'on effectue le traitement à une température de 10 à 90°C.

6. Procédé selon l'une quelconque des revendications 1 à 3, caractérisé en ce qu'on effectue le traitement à une température de 60 à 80°C.

7. Procédé selon l'une quelconque des revendications 1 à 6, caractérisé en ce qu'on fait réagir le fluorhydrate complexe (I) en discontinu avec l'alcène halogéné (II) et on sélectionne en l'occurrence le rapport molaire alcène halogéné (II):fluorhydrate d'amine complexe (I) au début de leur mise en réaction de telle sorte que l'amine contenue dans le fluorhydrate (I) se sépare sous forme d'une phase liquide supplémentaire à concurrence de 5 à 90%, et on sépare l'amine.

8. Procédé selon la revendication 7, caractérisé en ce qu'on sélectionne le rapport molaire alcène halogéné (II):fluorhydrate d'amine complexe (I) de telle sorte que l'amine se sépare à concurrence de 10 à 90%.

9. Procédé selon l'une quelconque des revendications 1 à 6, caractérisé en ce qu'on effectue le traitement du fluorhydrate complexe (I) par mise en réaction avec un alcène halogéné (II) sous forme gazeuse dans un réacteur allongé, par exemple une colonne à bulles, et on sépare l'amine précipitée sous forme d'une phase séparée.

10. Procédé selon la revendication 9, caractérisé en ce qu'on sépare en continu l'amine qui précipite, dans la mesure de sa formation, et on la remplace par une quantité équivalente de fluorhydrate d'amine complexe (I).
